# EUROPEAN PATENT APPLICATION

(11) **EP 1 523 989 A1**
(43) Date of publication of application: **20.04.2005**
(21) Application number: 03023500.6
(22) Date of filing: 18.10.2003
(51) Int. Cl.: A61K 39/00, A61P 35/00

(54) **Anti cancer vaccine derived from autologous plasma or leucocytes**

(71) Applicant: Kraja, Shuajp J., Shkoder (AL)
(72) Inventor: Kraja, Shuajp J., Shkoder (AL)
(74) Representative: Jönsson, Hans-Peter, Dr.Dipl.-Chem.

(57) **Abstract**

The present invention is related to a process for the preparation of a human anti cancer vaccine derived from autologous serum or leukocytes for the treatment of patients suffering from cancer.

## Description

The present invention is related to a process for the preparation of a human anti cancer vaccine and the treatment of patients suffering from cancer.

In the past the common treatment of cancer is dominated by chemo- or radio-therapy with all the side effects commonly known to public.

Thus, there is an urgent need for new and successful treatment of cancer.

The present invention deals with a new anti cancer vaccine which has been prepared in particular by a specific process for the preparation thereof.

Thus, in a first embodiment the present invention is related to a process for the preparation of a human anti cancer vaccine treating blood serum of a patent suffering from cancer with formaldehyde.

In a further embodiment, the invention relates to such a process containing the following steps:
(a) extracorporal coagulating a defined amount of human blood of a patient suffering from cancer, who has not been treated by chemo- or radiotherapy,
(b) centrifuging said blood in centrifuge at a rotational speed of at least 2000 rpm for at least 30 sec,
(c) combining the supernatant serum with an aqueous solution of formaldehyde at a temperature of at least 37 °C for at least 28 days,
(d) treating the product of step (c) with an aqueous solution of phosphoric acid,
(e) filtering the product of step (d),
(f) treating the precipitate of step (e) with an aqueous solution of hydrochloric acid by lowering the pH value to at least 4,
(g) centrifuging the product of step (f) at a rotational speed of at least 2000 rpm,
(h) collecting the precipitate of step (g),
(i) treating and solving the precipitate of step (h) with a buffer to raise the pH to a physiological value and
(j) repeating steps (d), (e), (g), (h), and (i) at least once.

In a second embodiment, the present invention relates to a process for the preparation of a human anticancer vaccine by treating vital lymphocytes of a person not suffering from cancer having the blood group 0, rhesus factor + to enzyme restriction to remove the DNA from the cells and combining said cells with vital lymphocytes of a patient suffering from cancer.

In a preferred embodiment, the present invention relates to a process, characterized in that the same amounts of said lymphocytes are combined.

In a further embodiment the invention is related to a different process for the preparation of a human anti cancer vaccine containing the following steps under sterile conditions:
(a) extracorporal treating a defined amount of human blood of a patient suffering from cancer, who has not been treated by chemo- or radiotherapy with a defined amount of heparin while shaking,
(b) diluting the product of step (a) containing vital lymphocytes with a defined amount of water while shaking,
(c) treating said lymphocytes (b) with an aqueous solution of sodium chloride,
(d) centrifuging said lymphocytes (c) at a rotational speed of at least 300 rpm for at least 10 min.,
(e) rinsing said precipitated lymphocytes with a buffer (PBS) and again repeating step (d) at least once,
(f) optionally checking vitality of the lymphocytes,
(g) centrifuging said lymphocytes of step (f) at a rotational speed of 400 rpm for at least 5 minutes,
(h) rinsing the precipitate with a buffer and again repeating step (g),
(i) storing a defined amount of human blood of a person having the blood group 0, rhesus factor + not suffering from cancer at a temperature of at least 37 °C for at least 60 hrs,
(j) separating the lymphocytes
(k) removing the DNA from said lymphocytes by enzyme restriction,
(I) mixing the same amounts of the product of step (h) and step (k),
(m) cultivating the mixture of step (e) at a temperature of at least 37 °C for at least 60 hrs,
(n) treating the product of step (m) in an ultra sonificator,
(o) centrifuging the product of step (n) at a rotational speed of at least 1000 rpm for at least 5 min,
(p) removing the precipitate,
(q) treating the supernatant with an aqueous solution of hydrochloric acid and thereby lowering the pH to a value of 3,5 to 4,
(r) centrifuging the product of step (g) at a rotational speed of at least 1000 rpm for at least 10 min,
(s) treating and solving the precipitate of step (r) with a buffer to raise the pH to a physiological value and
(t) repeating steps (r) and (s) at least once to obtain the desired vaccine.

After several years of research and development with several patients the inventor has come to the conclusion that the above referenced process leads to extraordinary results of a complete healing of patients suffering from different kinds of tumors.

The processes for the preparation of the vaccine are characterized by two different methods, whereby for the first method cells of a patient himself are used whereas for the second method of preparation the cells obtained are combined with healthy cells of another person. Thus, in both cases cells of patients suffering from tumors are used.

The cells taken from the patients are extracorporal manipulated in the laboratory by isolating the lymphocytes of the cancer cells to prepare cultures for the breeding of the cells.

The time necessary for such breeding is about 4 weeks and thus, the cells are stored in a fertile environment. During this time they are biochemically manipulated to obtain a hyper immunized antigen which may be used in particular as vaccine for several cancer types, in particular lung-cancer, breast-cancer and Hodgkin-lymphome.

Once the vaccine has been prepared it is administered to the patient himself originating the cells whereas in the second case said vaccine may be injected to any patient suffering from cancer.

Whereas in the first case of manipulation and administration to a patient optimal results are received without contra-indication with respect to the blood group, the second variation of the process of the preparation of the vaccine requires the measurement of blood compatibility.

The vaccines according to both major embodiments are injected subcutaneous in an amount of 1 to 2 cm³ depending on the development of the decease. The vaccine is administered three times within 28 days. Six months after the first treatment the treatment is repeated with the same dosage each with 1 to 2 cm³ for enhancing the development of gene immunity. Again, one year after the first treatment the treatment is repeated by applying the vaccine two times within 28 days. At the end of the procedure the patient is completely healed.

After the administration of the second injection of the vaccine, one observes a reduction of the malicious tumor whereas during the following treatment one observes the total disappearance of the tumor mass.

### Examples of the Invention:

### First Method:

50 ml blood are taken from a patient suffering from cancer for the preparation of blood platelets. It has to be ensured that said patient has not been treated by radio- or chemo-therapy in advance.

25 ml of said blood are coagulated by a standard erythrocyte sedimentation rate procedure. From this blood the serum is taken and centrifuged for 1 min at a rotational speed of 3000 rpm. The supernatant is treated with an aqueous solution of formaldehyde having a concentration of 35 % formaldehyde in an amount of 4 ml/l serum. Said reaction mixture is held at a temperature of 38 °C for 28 days allowing the preparation of an antitoxin. Said antitoxin is treated with an aqueous solution of phosphoric acid (m-HPO₃) (20 % by weight) in an amount of 10 ml/l per I anatoxin.

By addition an aqueous solution of hydrochloric acid (37 % by weight) the pH value is raised to 3.5 to 4. Said antitoxin again is centrifuged for 15 min at a rotational speed of 2000 rpm.

The precipitate is dissolved in a buffer (PBS) to adjust the pH at around 7 to 7.4. In order to purify the antitoxin, the solution again is treated with phosphoric acid (20 % by weight) by raising the pH to about 4.0. Again the precipitate is dissolved in said buffer whereby the pH is raised to 7 to 7.4.

Said procedure is repeated 2 to 3 times yielding in the immunogen of said antitoxin with a high imogene potential.

### Second Method:

25 ml blood according to example 1 is treated with 10 units standard Heparine per 1 ml blood. Said blood can be stored in a container at a temperature of liquid nitrogen for several months and taken when necessary. One part of said blood containing vital lymphocytes is diluted with 5 parts of distilled water. During the following period of 15 sec said blood is shaken vigorously. Immediately thereafter said liquid is added to 125 ml of a 1.7 % solution of Nacl. The complete reaction mixture is centrifuged for 20 min at a rotational speed of 400 rpm. At the end of said step a precipitate containing vital lymphocytes having a homogeneous color (silver) has been produced. It is necessary to check the vitality of said lymphocytes by routine determination.

The supernatant is removed and the precipitate is rinsed with a buffer (PBS) and again centrifuged for 10 min at a rotational speed of 400 rpm. Said rinsing procedure is repeated 2 to 3 times.

The vitality of said lymphocytes is checked by adding an aqueous solution of 1 % of trypan blue. The observation with a microscope reveals colored lymphocytes being dead and uncolored vital lymphocytes. It is self-evident that the complete procedure should be handled under sterile conditions. The amount of lymphocytes to be filled in a container for reaction should not exceed 2 cm³. The reaction mixture is held for 72 hours at a temperature of 37 °C by moving the reaction vessel approximately 30 times per hour. For breeding a standard culture medium is used. In particular, Eagle (MEM) containing serum human inactive 15 %, Fifth-hemagglutinin 0.01 ml, Kanamycine 1 % and heparin (40 units) for 1 ml.

Thereafter, the reaction product is centrifuged for 1 min at a rotational speed of 500 rpm. The supernatant is removed and the precipitate is rinsed with a buffer (PBS) again the rinsed precipitate is centrifuged for at least 10 min at a rotational speed of 600 rpm.

Half of said material is stored in a container under nitrogen atmosphere to allow a later reproduction. The other part of the reaction product is combined with the same amount of lymphocytes from a patient having the blood group 0, rhesus + who was diagnosed to be totally health. Said blood was stored at 37 °C for 72 h observing a change in the morphology of the lymphocytes.

The combined blood was treated by standard enzyme restriction to remove the DNA from the cells.

Said genetically manipulated material was mixed with the same amount of the reaction product stored in a container under nitrogen atmosphere.

The reaction mixture is stored for 72 h in a thermostat at 37 °C and thereafter treated in an ultra sonificator. Again the reaction product is centrifuged to remove the destroyed mass of the cells. The supernatant is taken by withdrawing the precipitate. The supernatant is dissolved in an aqueous solution of hydrochloric acid (37 %) by raising the pH to 3.5 to 4. Again the reaction product is centrifuged for 10 min at a rotational speed of 1500 rpm.

The above referenced removed precipitate is dissolved in a buffer (PBS 3 ml per 1 ml of the mass of the precipitate). Again, said precipitate is centrifuged at a rotational speed of 400 rpm for 1 min. Said procedure is repeated to 2 to 3 times yielding a hyper immune antigen.

Said antigen could be used for diagnostic purposes and as vaccine in therapy. In this respect, it is preferred to a combine said antigen with an aluminum phosphate gel allowing the uptake of the vaccine in the body in a retarded form.

### Therapeutical Application:

For the treatment of patients suffering from cancer, two types of vaccines have been prepared:
a) auto-vaccine according to the first embodiment of the invention an aliquot mixture of said antitoxin vaccine + lymphocyte vaccine for the modified genetic material was injected to one person;
b) hetero-vaccine the difference according to the second embodiment of the invention was taken from several persons and thus, it could be used for all types of cancer.

### Medical Treatment of the Patient with vaccine:

After preparation of the vaccine according to the first and second method three doses of 1 ml vaccine were given to the patients by subcutaneous injection each month. Accordingly, the treatment of the patient took at least 3 months. 6 months after the first treatment said patient was again treated with 1 ml vaccine. One year after the first treatment, the patient was again treated with 2 doses of 1 ml within 1 month.

## Claims

1. Process for the preparation of a human anti cancer vaccine **characterized by** treating blood serum of a patent suffering from cancer with formaldehyde.

2. Process according to claim 1 containing the following steps:
(a) extracorporal coagulating a defined amount of human blood of a patient suffering from cancer, who has not been treated by chemo- or radiotherapy,
(b) centrifuging said blood in centrifuge at a rotational speed of at least 2000 rpm for at least 30 sec,
(c) combining the supernatant serum with an aqueous solution of formaldehyde at a temperature of at least 37 °C for at least 28 days,
(d) treating the product of step (c) with an aqueous solution of phosphoric acid,
(e) filtering the product of step (d),
(f) treating the precipitate of step (e) with an aqueous solution of hydrochloric acid by lowering the pH value to at least 4,
(g) centrifuging the product of step (f) at a rotational speed of at least 2000 rpm,
(h) collecting the precipitate of step (g),
(i) treating and solving the precipitate of step (h) with a buffer to raise the pH to a physiological value and
(j) repeating steps (d), (e), (g), (h), and (i) at least once.

3. Process for the preparation of a human anticancer vaccine **characterized by**
treating vital lymphocytes of a person not suffering from cancer having the blood group 0, rhesus factor + to enzyme restriction, thereby removing the DNA from the cells and combining said cells with vital lymphocytes of a patient suffering from cancer.

4. Process according to claim 3, characterized that the same amounts of said lymphocytes are combined.

5. Process according to claim 3 or 4, containing the following steps under sterile conditions:
(a) extracorporal treating a defined amount' of human blood of a patient suffering from cancer, who has not been treated by chemo- or radiotherapy with a defined amount of heparin while shaking,
(b) diluting the product of step (a) containing vital lymphocytes with a defined amount of water while shaking,
(c) treating said lymphocytes (b) with an aqueous solution of sodium chloride,
(d) centrifuging said lymphocytes (c) at a rotational speed of at least 300 rpm for at least 10 min.,
(e) rinsing said precipitated lymphocytes with a buffer (PBS) and again repeating step (d) at least once,
(f) optionally checking vitality of the lymphocytes,
(g) centrifuging said lymphocytes of step (f) at a rotational speed of 400 rpm for at least 5 minutes,
(h) rinsing the precipitate with a buffer and again repeating step (g),
(i) storing a defined amount of human blood of a person having the blood group 0, rhesus factor + not suffering from cancer at a temperature of at least 37 °C for at least 60 hrs,
(j) separating the lymphocytes
(k) removing the DNA from said lymphocytes by enzyme restriction,
(l) mixing the same amounts of the product of step (h) and step (k),
(m) cultivating the mixture of step (e) at a temperature of at least 37 °C for at least 60 hrs,
(n) treating the product of step (m) in an ultra sonificator,
(o) centrifuging the product of step (n) at a rotational speed of at least 1000 rpm for at least 5 min,
(p) removing the precipitate,
(q) treating the supernatant with an aqueous solution of hydrochloric acid and thereby lowering the pH to a value of 3,5 to 4,
(r) centrifuging the product of step (g) at a rotational speed of at least 1000 rpm for at least 10 min,
(s) treating and solving the precipitate of step (r) with a buffer to raise the pH to a physiological value and
(t) repeating steps (r) and (s) at least once to obtain the desired vaccine.

6. Anticancer vaccine obtainable by a process as defined in anyone of claims 1 to 5.
